# EUROPEAN PATENT APPLICATION

(11) **EP 3 184 635 A1**
(43) Date of publication of application: **28.06.2017**
(21) Application number: 15833168.6
(22) Date of filing: 18.08.2015
(51) Int. Cl.: C12N 15/09, C12Q 1/68

(54) **CANCER MARKER AND CANCER DETERMINATION METHOD**

(30) Priority: 18.08.2014 JP 2014166249; 19.08.2014 JP 2014166457
(71) Applicant: Wako Pure Chemical Industries, Ltd., Osaka 540-8605 (JP)
(72) Inventor: HAYASHIDA Yukinobu, Amagasaki-shi Hyogo 661-0963 (JP)
(74) Representative: Winter, Brandl, Fürniss, Hübner, Röss, Kaiser, Polte - Partnerschaft mbB
(86) International application number: PCT/JP2015/073083
(87) International publication number: WO 2016/027794

(57) **Abstract**

The purpose of the present invention is to provide a method for determining cancer, and a cancer marker enabling determination of canceration of cells in good precision, and enabling determination of canceration of various cells. The present invention relates to "a cancer marker, consisting of a portion of or all of the nucleotide sequence represented by SEQ ID NO: 1 in a FOXB2 gene derived from a cell, and being continuous nucleotide sequences comprising at least the nucleotide sequence represented by SEQ ID NO: 3, wherein 30% or more of cytosines of the -CG- sequences present in said nucleotide sequence are methylated", "a cancer marker, consisting of a portion of or all of the nucleotide sequence represented by SEQ ID NO: 1 in a FOXB2 gene derived from a cell, and being continuous nucleic sequences comprising at least the nucleotide sequence represented by SEQ ID NO: 2, wherein 30% or more of cytosines of the -CG- sequences present in said nucleotide sequence are methylated", and "a method for determining canceration of a cell on the basis of methylation ratio, wherein said methylation ratio is measured by a bisulfite reaction for a portion of or all of cytosines of the -CG- sequences present in the nucleotide sequence represented by SEQ ID NO: 1 in a FOXB2 gene derived from a cell".

## Description

### TECHNICAL FIELD

The present invention relates to a cancer marker and a cancer determination method using the same.

### BACKGROUND ART

A group of transcription factors that has a forkhead or a winged helix DNA-binding domain is called FOX (Forkhead box), and about 50 kinds are present in human. Although they act mainly as an embryologic regulating factor, a tissue-specific regulating factor, and cell cycle regulating factor, there are many FOXes whose action is little elucidated. Among them, FOXB2 has been reported to be involved in neurogenesis of embryos in African clawed frog, however, its function in mammals has not been elucidated.

On the other hand, methylation of DNA has been known to be associated with canceration, and in JP-A2008-283947, there has been described a method for detecting esophageal cancer, on the basis of methylation ratio in a CpG island (CpG dinucleotide: -CG- sequence) of a FOXB2 gene.

### CITATION LIST

### Patent Literature

Patent Literature 1: JP- A-2008-283947

### SUMMARY OF INVENTION

### TECHNICAL PROBLEM

In the Patent Literature 1, there has been described that cancer cells are often underwent methylation of cytosine of CpG dinucleotide, and further, there has been described that by measuring the FOXB2 gene of esophageal cancer by a BAMCA method, cytosine of CpG dinucleotide was confirmed to be methylated. However, because the BAMCA method measures methylation of CCCGGG sequence, the measurement point is very limited. On the other hand, the present inventors have discovered, as a result of extensive investigation on methylation of cytosine of CpG dinucleotide in cancer cells, that even in cancer cells, not all CpG dinucleotides are methylated, and in addition, methylation ratio was discovered to differ by position of CpG dinucleotide. Therefore, determination of cancer by methylation in several places, such as by the BAMCA method, has low precision, and thus a method for determining canceration of cells in higher precision has been desired.

In addition, since cancer occurs in many cell types, such a cancer determination method, that is applicable not only to canceration of a specific cell but also to various cells, has also been desired. Therefore, it is an object of the present invention to provide a cancer determination method, as well as a cancer marker which is capable of determining canceration of cells in good precision, and is capable of determining canceration of various cells.

### SOLUTION TO PROBLEM

The present inventors have investigated extensively in view of the above situation, and have discovered, as a result of analysis of a specific region of the FOXB2 gene, by a bisulfite method, using genomic DNA derived from various normal cells, blood of a healthy subject and various cancer cells, as a template, cytosine of CpG dinucleotide in the specific region was in an unmethylated state in the genomic DNA derived from various normal cells and the whole blood of the healthy subject, and those derived from various cancer cells were in a methylated state. Thus, from this result, the present invention has been completed.

That is, the present invention relates to "a cancer marker consisting of a portion of or all of the nucleotide sequence represented by SEQ ID NO: 1 in a FOXB2 gene derived from a cell, and being continuous nucleotide sequences comprising at least the nucleotide sequence represented by SEQ ID NO: 3, wherein 30% or more of cytosines of the -CG- sequences present in said nucleotide sequence are methylated", "a cancer marker, consisting of a portion of or all of the nucleotide sequence represented by SEQ ID NO: 1 in a FOXB2 gene derived from a cell, and being continuous nucleotide sequences comprising at least the nucleotide sequence represented by SEQ ID NO: 2, wherein 30% or more of cytosines of the -CG- sequences present in said nucleotide sequence are methylated", and "a method for determining canceration of cells wherein methylation ratio for a portion of or all of cytosines of the -CG- sequences present in the nucleotide sequence represented by SEQ ID NO: 1 in a FOXB2 gene derived from a cell, is measured by a bisulfite reaction, and canceration of the cell is determined on the basis of methylation ratio.

### ADVANTAGEOUS EFFECTS OF INVENTION

By using the cancer marker of the present invention as an indicator, and by the method of the present invention, it is possible to determine canceration of cells for various cells regardless of the kind of cells. Furthermore, since the method of the present invention measures methylation ratio of cytosine of CpG dinucleotide of a specific region, it is possible to determine canceration of cells in good precision.

### BRIEF DESCRIPTION OF DRAWINGS

Fig. 1 is a diagram with respect to the FOXB2 gene of hiPS (normal human skin fibroblast cells) of Example 1, showing whether cytosine of CpG dinucleotide is unmethylated cytosine or methylated cytosine.
Fig. 2 is a diagram with respect to the FOXB2 gene of HDF (normal human skin fibroblast cells) of Example 1, showing whether cytosine of CpG dinucleotide is unmethylated cytosine or methylated cytosine.
Fig. 3 is a diagram with respect to the FOXB2 gene of WI-38 (normal human lung fibroblast cells) of Example 1, showing whether cytosine of CpG dinucleotide is unmethylated cytosine or methylated cytosine.
Fig. 4 is a diagram with respect to the FOXB2 gene of CCD-8Lu (normal human lung diploid cells) of Example 1, showing whether cytosine of CpG dinucleotide is unmethylated cytosine or methylated cytosine.
Fig. 5 is a diagram with respect to the FOXB2 gene of HE23 (normal human fetal cells) of Example 1, showing whether cytosine of CpG dinucleotide is unmethylated cytosine or methylated cytosine.
Fig. 6 is a diagram with respect to the FOXB2 gene in the whole blood of Example 1, showing whether cytosine of CpG dinucleotide is unmethylated cytosine or methylated cytosine.
Fig. 7 shows the nucleotide sequence of a FOXB2 promoter region (PCR amplification region, GenBank Accession No. AL353637: 106566-107058, SEQ ID NO: 10).
Fig. 8 is a diagram with respect to the FOXB2 gene of IMR-32 (human abdominal neuroblastoma cells) of Example 2, showing whether cytosine of CpG dinucleotide is unmethylated cytosine or methylated cytosine.
Fig. 9 is a diagram with respect to the FOXB2 gene of HEK293 (human embryonic kidney cells) of Example 2, showing whether cytosine of CpG dinucleotide is unmethylated cytosine or methylated cytosine.
Fig. 10 is a diagram with respect to the FOXB2 gene of HeLa (human cervical cancer cells) of Example 2, showing whether cytosine of CpG dinucleotide is unmethylated cytosine or methylated cytosine.
Fig. 11 is a diagram with respect to the FOXB2 gene of HepG2 (human hepatoma cells) of Example 2, showing whether cytosine of CpG dinucleotide is unmethylated cytosine or methylated cytosine.
Fig. 12 is a diagram with respect to the FOXB2 gene of MDA-MB-231 (human breast cancer) of Example 2, showing whether cytosine of CpG dinucleotide is unmethylated cytosine or methylated cytosine.
Fig. 13 is a diagram with respect to the FOXB2 gene of TYK-nu (human ovarian undifferentiated cancer cell) of Example 2, showing whether cytosine of CpG dinucleotide is unmethylated cytosine or methylated cytosine.
Fig. 14 is a diagram with respect to the FOXB2 gene of HCC2998 (human large intestine adenocarcinoma cells) of Example 2, showing whether cytosine of CpG dinucleotide is unmethylated cytosine or methylated cytosine.
Fig. 15 is a diagram with respect to the FOXB2 gene of HCT-15 (human colon adenocarcinoma cells) of Example 2, showing whether cytosine of CpG dinucleotide is unmethylated cytosine or methylated cytosine.
Fig. 16 is a diagram with respect to the FOXB2 gene of HCT-116 (human colon adenocarcinoma cells) of Example 2, showing whether cytosine of CpG dinucleotide is unmethylated cytosine or methylated cytosine.
Fig. 17 is a diagram with respect to the FOXB2 gene of LoVo (human colon adenocarcinoma cells (clavicle lymph node)) of Example 2, showing whether cytosine of CpG dinucleotide is unmethylated cytosine or methylated cytosine.
Fig. 18 is a diagram with respect to the FOXB2 gene of SW620 (human colon adenocarcinoma cells) of Example 2, showing whether cytosine of CpG dinucleotide is unmethylated cytosine or methylated cytosine.
Fig. 19 is a diagram with respect to the FOXB2 gene of SW837 (human colorectal adenocarcinoma cells) of Example 2, showing whether cytosine of CpG dinucleotide is unmethylated cytosine or methylated cytosine.
Fig. 20 is a diagram with respect to the FOXB2 gene of COLO 205 (human colon adenocarcinoma cells) of Example 2, showing whether cytosine of CpG dinucleotide is unmethylated cytosine or methylated cytosine.
Fig. 21 is a diagram with respect to the FOXB2 gene of WiDr (human colon adenocarcinoma cells) of Example 2, showing whether cytosine of CpG dinucleotide is unmethylated cytosine or methylated cytosine.
Fig. 22 is a diagram with respect to the FOXB2 gene of LNCap. Clone FGC (human prostate cancer cells) of Example 2, showing whether cytosine of CpG dinucleotide is unmethylated cytosine or methylated cytosine.
Fig. 23 is a diagram with respect to the FOXB2 gene of NCI-H460 (human non-small cell lung cancer cells) of Example 2, showing whether cytosine of CpG dinucleotide is unmethylated cytosine or methylated cytosine.
Fig. 24 is a diagram with respect to the FOXB2 gene of ACHN (human renal adenocarcino cells) of Example 2, showing whether cytosine of CpG dinucleotide is unmethylated cytosine or methylated cytosine.
Fig. 25 is a diagram with respect to the FOXB2 gene of MCF-7 (human breast cancer cells) of Example 2, showing whether cytosine of CpG dinucleotide is unmethylated cytosine or methylated cytosine.
Fig. 26 is a diagram with respect to the FOXB2 gene of PANC-1 (human pancreatic adenocarcinoma cells) in Example 2, showing whether cytosine of CpG dinucleotide is unmethylated cytosine or methylated cytosine.
Fig. 27 is a diagram with respect to the FOXB2 gene of CFPAC-1 (human pancreatic adenocarcinoma) of Example 2, showing whether cytosine of CpG dinucleotide is unmethylated cytosine or methylated cytosine.
Fig. 28 is a diagram with respect to the FOXB2 gene of DAUDI (human Burkitt's lymphoma cells) of Example 2, showing whether cytosine of CpG dinucleotide is unmethylated cytosine or methylated cytosine.
Figure 29 is a diagram with respect to the FOXB2 gene of HL60 (human promyelocytic leukemi cells) of Example 2, showing whether cytosine of CpG dinucleotide is unmethylated cytosine or methylated cytosine.
Fig. 30 is a diagram with respect to the FOXB2 gene of Jurkat (human T cell leukemi cells) of Example 2, showing whether cytosine of CpG dinucleotide is unmethylated cytosine or methylated cytosine.
Figure 31 is a diagram with respect to the FOXB2 gene of Mono Mac 6 (human monocytic leukemi cells) of Example 2, showing whether cytosine of CpG dinucleotide is unmethylated cytosine or methylated cytosine.
Fig. 32 is a diagram with respect to the FOXB2 gene of THP-1 (human acute monocytic leukemi cells) of Example 2, showing whether cytosine of CpG dinucleotide is unmethylated cytosine or methylated cytosine.

### DESCRIPTION OF EMBODIMENTS

### [The cancer marker of the present invention]

The cancer marker of the present invention is the one consisting of a portion of or all of the nucleotide sequence represented by SEQ ID NO: 1, present in a promotor region of the FOXB2 gene derived from cells, and comprising at least the nucleotide sequence represented by SEQ ID NO: 3, or comprising at least the nucleotide sequence represented by SEQ ID NO: 2 (hereinafter, they may be abbreviated sometimes as the cancer marker nucleotide sequence pertaining to the present invention), in which 30% or more, preferably 40% or more, and more preferably 50% or more of cytosines of the -CG- sequences present in the nucleotide sequence are methylated.

The cancer marker nucleotide sequence pertaining to the present invention includes, specifically, the nucleotide sequence represented by SEQ ID NO: 1; the nucleotide sequence represented by SEQ ID NO: 2; the nucleotide sequence represented by SEQ ID NO: 3; continuous nucleotides consisting of the nucleotide sequence represented by SEQ ID NO: 2 and at least one nucleotide adjacent to SEQ ID NO: 2 out of 1 to 72 nucleotides adjacent to the 3' side thereof; continuous nucleotides consisting of the nucleotide sequence represented by SEQ ID NO: 2 and at least one nucleotide adjacent to SEQ ID NO: 2 out of 1 to 21 nucleotides adjacent to the 5' side thereof; continuous nucleotides consisting of the nucleotide sequence represented by SEQ ID NO: 2 and at least one nucleotide adjacent to SEQ ID NO: 2 out of 1 to 72 nucleotides adjacent to the 3' side thereof and at least one nucleotide adjacent to SEQ ID NO: 2 out of 1 to 21 nucleotides adjacent to the 5' side of SEQ ID NO: 2 (excluding the nucleotide sequence represented by SEQ ID NO: 1); continuous nucleotides consisting of the nucleotide sequence represented by SEQ ID NO: 3 and at least one nucleotide adjacent to SEQ ID NO: 3 out of 1 to 72 nucleotides adjacent to the 3' side thereof; continuous nucleotides consisting of the nucleotide sequence represented by SEQ ID NO: 3 and at least one nucleotide adjacent to SEQ ID NO: 3 out of 1 to 56 nucleotides adjacent to the 5' side thereof; continuous nucleotides consisting of the nucleotide sequence represented by SEQ ID NO: 3 and at least one nucleotide adjacent to SEQ ID NO: 3 out of 1 to 72 nucleotides adjacent to the 3' side thereof and at least one nucleotide adjacent to SEQ ID NO: 3 out of 1 to 56 nucleotides adjacent to the 5' side of SEQ ID NO: 3 (excluding the nucleotide sequence represented by SEQ ID NO: 1); or the like.

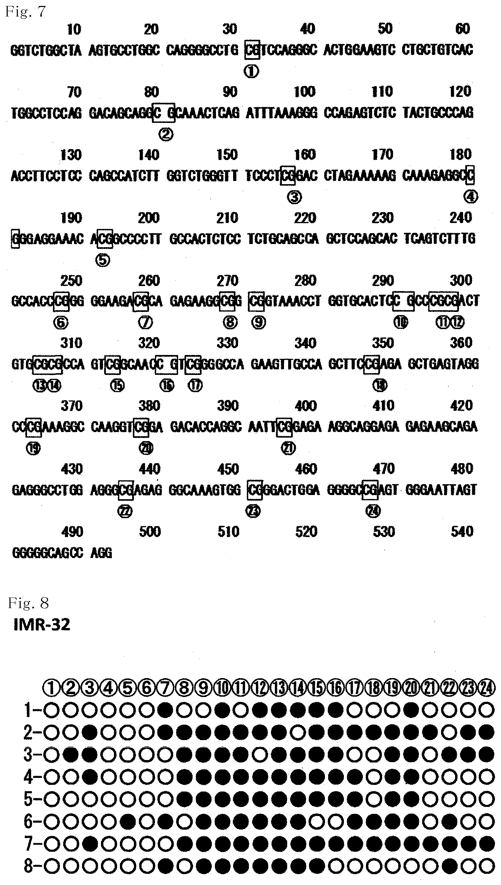

The cancer marker nucleotide sequence pertaining to the present invention includes, more specifically, the nucleotide sequence represented by SEQ ID NO: 1 (in Fig. 7, the nucleotide sequences of circle 6 to circle 21); the nucleotide sequence represented by SEQ ID NO: 2 (in Fig. 7, the nucleotide sequences of circle 8 to circle 17); the nucleotide sequence represented by SEQ ID NO: 3 (in Fig. 7, the nucleotide sequences of circle 13 to circle 17); a continuous nucleotide sequence consisting of the nucleotide sequence represented by SEQ ID NO: 2, and 23 nucleotides, 40 nucleotides, 54 nucleotides, or 72 nucleotides adjacent to the 3' side of the nucleotide sequence (in Fig. 7, the nucleotide sequences of circle 8 to circle 18, the nucleotide sequences of circle 8 to circle 19, the nucleotide sequences of circle 8 to circle 20, or the nucleotide sequences of circle 8 to circle 21); a continuous nucleotide sequence consisting of the nucleotide sequence represented by SEQ ID NO: 2 and 11 nucleotides or 21 nucleotides adjacent to the 5' side of the nucleotide sequence (in Fig. 7, the nucleotide sequences of circle 7 to circle 17, or the nucleotide sequences of circle 6 to circle 17); a continuous nucleotide sequence consisting of the nucleotide sequence represented by SEQ ID NO: 2, and 23 nucleotides, 40 nucleotides, 54 nucleotides, or 72 nucleotides adjacent to the 3' side of the nucleotide sequence, and 11 nucleotides or 21 nucleotides adjacent to the 5' side of the nucleotide sequence represented by SEQ ID NO: 2 (in Fig. 7, the nucleotide sequences of circle 7 to circle 18, the nucleotide sequences of circle 7 to circle 19, the nucleotide sequences of circle 7 to circle 20, the nucleotide sequences of circle 7 to circle 21, the nucleotide sequences of circle 6 to circle 18, the nucleotide sequences of circle 6 to circle 19, the nucleotide sequences of circle 6 to circle 20, or the nucleotide sequences of circle 6 to circle 21); a continuous nucleotide sequence consisting of the nucleotide sequence represented by SEQ ID NO: 3, and 23 nucleotides, 40 nucleotides, 54 nucleotides, or 72 nucleotides adjacent to the 3' side of the nucleotide sequence (in Fig. 7, the nucleotide sequences of circle 13 to circle 18, the nucleotide sequences of circle 13 to circle 19, the nucleotide sequences of circle 13 to circle 20, or the nucleotide sequences of circle 13 to circle 21); a continuous nucleotide sequence consisting of the nucleotide sequence represented by SEQ ID NO: 3, and 8 nucleotides, 10 nucleotides, 14 nucleotides, 32 nucleotides, 35 nucleotides, 46 nucleotides, or 56 nucleotides adjacent to the 5' side of the nucleotide sequence (in Fig. 7, the nucleotide sequences of circle 12 to circle 17, the nucleotide sequences of circle 11 to circle 17, the nucleotide sequences of circle 10 to circle 17, the nucleotide sequences of circle 9 to circle 17, the nucleotide sequences of circle 8 to circle 17, the nucleotide sequences of circle 7 to circle 17, or the nucleotide sequences of circle 6 to circle 17); a continuous nucleotide sequence consisting of the nucleotide sequence represented by SEQ ID NO: 3, and 23 nucleotides, 40 nucleotides, 54 nucleotides, or 72 nucleotides adjacent to the 3' side of the nucleotide sequence, and 8 nucleotides, 10 nucleotides, 14 nucleotides, 32 nucleotides, 35 nucleotides, 46 nucleotides, or 56 nucleotides adjacent to the 5' side of the nucleotide sequence represented by SEQ ID NO: 3 (in Fig. 7, the nucleotide sequence having circle 6 to circle 12 as the 5'-terminal, and circle 18 to circle 21 as the 3'-terminal); or the like. Among them, the nucleotide sequence represented by SEQ ID NO: 1, the nucleotide sequence represented by SEQ ID NO: 2, the nucleotide sequence represented by SEQ ID NO: 3, and the like, are preferable, and the nucleotide sequence represented by SEQ ID NO: 2, the nucleotide sequence represented by SEQ ID NO: 3, and the like, are more preferable, and the nucleotide sequence represented by SEQ ID NO: 3 is further preferable.

It should be noted that, since the cancer marker nucleotide sequence pertaining to the present invention can be used as the same cancer marker, as long as cytosine of the -CG- sequence contained in the sequence is the same, the specific nucleotide sequence may contain a portion of or all of the nucleotide sequence up to one nucleotide before cytosine of the next -CG-sequence toward the 5'-terminal or/and toward the 3'-terminal. That is, for example, the nucleotide sequence represented by SEQ ID NO: 1 may comprise at least one nucleotide adjacent to SEQ ID NO:1 out of 53 nucleotides adjacent to the 5' side or/and at least one nucleotide adjacent to SEQ ID NO:1 out of 38 nucleotides adjacent to the 3' side. For example, the nucleotide sequence represented by SEQ ID NO: 2 may comprise at least one nucleotide adjacent to SEQ ID NO: 2 out of 10 nucleotides adjacent to the 5' side or/and at least one nucleotide adjacent to SEQ ID NO: 2 out of 22 nucleotides adjacent to the 3' side. For example, the nucleotide sequence represented by SEQ ID NO: 3 may comprise at least one nucleotide adjacent to SEQ ID NO: 3 out of 7 nucleotides adjacent to the 5' side or/and at least one nucleotide adjacent to SEQ ID NO: 3 out of 22 nucleotides adjacent to the 3' side.

In the case where the cancer marker nucleotide sequence pertaining to the present invention is the nucleotide sequence represented by SEQ ID NO: 2 or the nucleotide sequence represented by SEQ ID NO: 3, cytosines of the -CG- sequence in the cancer marker are the one in which 40% or more, preferably 50% or more, and more preferably 60% or more thereof is methylated.

The nucleotide sequence represented by the SEQ ID NO: 1 has been described in the nucleotide numbers 106812-106961 of Genbank Accession No. AL353637. The nucleotide sequence represented by the SEQ ID NO: 2 has been described as nucleotide numbers 106833-106889 in Genbank Accession No. AL353637. The nucleotide sequence represented by the SEQ ID NO: 3 has been described as nucleotide numbers 106868-106889 in Genbank Accession No. AL353637.

The nucleotide sequences represented by SEQ ID NO: 1 and 2 encompass the one in which deletion, replacement, or addition of nucleotide has occurred by mutations caused by species difference of organism, individual difference, or cell or tissue difference.

The FOXB2 gene in the cancer markers of the present invention may be the one derived from cells, and the type of the cells is selected in response to cancer to be determined. For example, in the case of determining liver cancer, gene derived from hepatic cells may be used, and in the case of determining prostate cancer, gene derived from prostate cells may be used. In addition, when canceration of leukocyte is determined, by separation of leucocytes from blood, or by using blood as it is, gene derived from leukocytes may be used. Cells from which the FOXB2 gene are derived are preferably derived from mammal, and those derived from human are particularly preferable. Cells to be used suitably include, for example, neuroblast, kidney, uterus, liver, mammary gland, ovary, large intestine (colon, rectum), prostate, lung, pancreas, bone marrow cells, T cells, and the like.

Methylation ratio of cytosine of the -CG- sequence present in the cancer marker nucleotide sequence pertaining to the present invention is calculated on the basis of number of methylated cytosine of the -CG-sequence, which is obtained by subjecting the cancer marker nucleotide sequence pertaining to the present invention to a bisulfite reaction, and detecting the methylated cytosine of the -CG- sequence. When the cancer marker nucleotide sequence pertaining to the present invention is subjected to the bisulfite reaction, unmethylated cytosine is converted to uracil, but methylated cytosine is not converted to uracil. Therefore, by analyzing the nucleotide sequence after the bisulfite reaction, and by comparing it with the normal nucleotide sequence, methylated cytosine can be detected, and by dividing number of methylated cytosine in the -CG- sequence by number of cytosine in the -CG- sequence, it is possible to calculate methylation ratio. It should be noted that, details of the bisulfite reaction will be described later in the section of the determination method for canceration of the present invention.

The cancer markers of the present invention can be obtained, for example, by obtaining the cancer marker nucleotide sequence pertaining to the present invention from the genomic DNA, after obtaining the genomic DNA from mammalian cells.

The method for obtaining the genomic DNA from the cells can be carried out, for example, according to a well-known DNA extraction method, such as an alkaline SDS method described in "Labo Manual for Genetic Engineering" (Maruzen Co., Ltd.), and "Handbook of Gene Technology" (Yodosha Co., Ltd.), or the like, or by extracting it from cells, microorganisms, viruses, and the like, using a commercially available extraction kit of the genomic DNA.

The method for obtaining the cancer marker nucleotide sequence pertaining to the present invention, from the genomic DNA obtained above, may be a method known per se, usually used in this field, and there is included, for example, a method for obtaining it, by the PCR, after designing primers capable of obtaining the objective nucleotide sequence, or the like. The primers to be used in this case include, for example, the followings.

Forward primer:
   GGTTTGGTTAAGTGTTTGGTTAGGGG (SEQ ID NO: 4)
   GTAGTTAGTTTTAGTATTTAGTTTTTGG (SEQ ID NO: 5)
Reverse primer:
   CCTAACTACCCCCACTAATTCCCACTC (SEQ ID NO: 6)
   CCCTCTCTACTTCTCTCTCCTACCTTCTC (SEQ ID NO: 7)

A specific method of the PCR may be carried out according to the PCR reaction described in the section of [Acquisition of DNA having the objective nucleotide sequence pertaining to the present invention] to be described later.

### [The determination method for canceration of the present invention]

The determination method for canceration of the present invention is carried out by measuring, by the bisulfite reaction, methylation ratio of a portion of or all of cytosines of the -CG- sequence present in the nucleotide sequence represented by SEQ ID NO: 1 in the FOXB2 gene derived from cells (hereinafter, sometimes, abbreviated as the objective nucleotide sequence pertaining to the present invention), and determining canceration of cells on the basis of the methylation ratio. Specifically, in the case where the methylation ratio is 30% or more, preferably 40% or more, and more preferably 50% or more, it is determined that cells are cancerous.

The objective nucleotide sequence pertaining to the present invention includes the same ones as the cancer marker nucleotide sequence pertaining to the present invention, and among them, the nucleotide sequence represented by SEQ ID NO: 2, and the nucleotide sequence represented by SEQ ID NO: 3 are preferable, and the nucleotide sequence represented by SEQ ID NO: 2 is more preferable.

When measuring methylation ratio of a portion of cytosines of the -CG- sequences present in the objective nucleotide sequence pertaining to the present invention, cytosines of the -CG- sequences to be used for determination of cancer are selected as appropriate, and methylation ratio of these cytosines may be measured. Combinations of cytosines of the -CG- sequences to be used for determination of cancer include, for example, in Fig. 7, a combination of circle 7 to circle 16, circle 7 to circle 17, circle 8 to circle 16, circle 8 to circle 17, circle 13 to circle 16 , circle 13 to circle 17, circle 7 to circle 10 and circle 13 to circle 16; a combination of circle 7 to circle 10 and circle 13 to circle 17; a combination of circle 8 to circle 10 and circle 13 to circle 16; and a combination of circle 8 to circle 10 and circle 13 to circle 17; and among them, circle 8 to circle 17 (corresponding to the nucleotide sequence represented by SEQ ID NO: 2), circle 13 to circle 17 (corresponding to the nucleotide sequence represented by SEQ ID NO: 3), and the like, are preferable, and circle 13 to circle 17, and the like, are more preferable. When methylation of cytosines of the -CG-sequences of the circle 8 to circle 17 or circle 13 to circle 17 is measured, or in the case where a portion of the nucleotide sequence represented by SEQ ID NO: 1 is the nucleotide sequence represented by SEQ ID NO: 2 or the nucleotide sequence represented by SEQ ID NO: 3, in the determination method for canceration of the present invention, it is determined that cells are cancerous when methylation ratio of cytosines of the -CG- sequences present in the nucleotide sequence is 40% or more, preferably 50% or more, and more preferably 60% or more.

In the determination method for canceration of the present invention, specifically, DNA having the objective nucleotide sequence pertaining to the present invention is obtained, after subjecting the genomic DNA of the FOXB2 gene derived from cells to the bisulfite reaction. After that, methylation ratio for all of cytosines of the -CG- sequences present in the nucleotide sequence is measured, or, a portion of cytosines of the -CG-sequences present in the nucleotide sequence is selected as described above, and methylation ratio of the selected cytosines is measured. Then canceration of cells is determined based on the resulting methylation ratio.

### Acquisition of the genomic DNA

In the determination method for canceration of the present invention, the genomic DNA of the FOXB2 gene derived from cells can be obtained, for example, by a well-known DNA extraction method, such as alkaline SDS method, described in "Labo Manual for Genetic Engineering" (Maruzen Co., Ltd.), and "Handbook of Gene Technology" (Yodosha Co., Ltd.), or the like, or by extracting it from the objective cells using a commercially available genomic DNA extraction kit. The objective cells may be selected as appropriate, according to cancer to be determined. As for the cells, those derived from mammals are preferable, and those derived from human are particularly preferable. In addition, the cells may be either cultured cells or cells collected from mammals, and the collected cells may be either cells collected directly, or cells collected from body fluid, such as blood.

### [Bisulfite reaction]

The bisulfite reaction, in the determination method for canceration of the present invention, may be carried out according to a bisulfite method known per se, and specifically, it may be carried out, for example, by subjecting the genomic DNA obtained above to single strand formation treatment, and after sulfonation of cytosine by a reaction with a bisulfite, making sulfonated cytosine hydrolyzed, and further desulfonated in the presence of an alkali. According to the reaction, methylated cytosine remains as it is without a reaction, and only non-methylated cytosine is converted to uracil.

The bisulfite reaction may be carried out in the presence of at least one kind of compounds shown by the following general formulae [1] to [8] described in WO2013/089063. Specific examples of substituents (R₁ to R₃₉) in these compounds, specific examples of the compounds, use amount of the compounds, a use method thereof, and the like, of that case, may be set and carried out according to the description of WO2013/089063.

The compound shown the general formula [1], 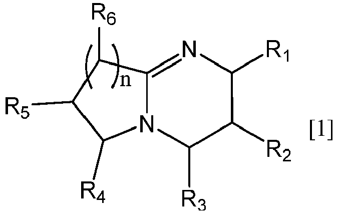 (wherein R₁ to R₆ each independently represent hydrogen or an alkyl group having 1 to 6 carbon atoms; and n represents an integer of 1 to 3.),
the compound shown by the general formula [2], 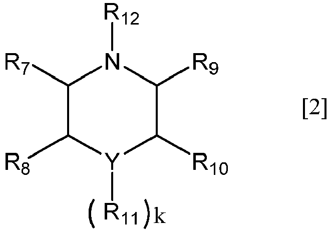 (wherein Y represents carbon atom, oxygen atom or nitrogen atom; R₇ to R₁₂ each independently represent hydrogen atom or an alkyl group having 1 to 6 carbon atoms; and k represents an integer of 0 to 2, provided that k represents 0 when Y is oxygen atom, k represents 1 when Y is nitrogen atom, and k represents 2 when Y is carbon atom.),
the compound represented by the general formula [3], 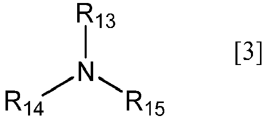 (wherein R₁₃ to R₁₅ each independently represent hydrogen atom, or an alkyl group having 1 to 6 carbon atoms.),
the compound represented by the general formula [4], 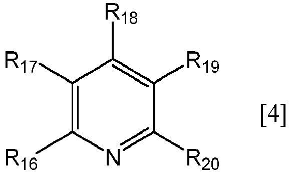 (wherein R₁₇ and R₁₉ each independently represent hydrogen atom, an amino group or an alkyl group having 1 to 6 carbon atoms; R₁₆, R₁₈ and R₂₀ each independently represent hydrogen atom, an amino group, an alkyl group having 1 to 6 carbon atoms, or a dialkylamino group having 2 to 6 carbon atoms; R₁₆ and R₁₇ may form benzene ring together with carbon atom adjacent to R₁₆, and carbon atom adjacent to R₁₇.),
the compound shown by the general formula [5], 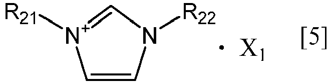 (wherein R₂₁ and R₂₂ each independently represent an alkyl group having 1 to 6 carbon atoms; and X₁ represents a counter anion.),
the compound represented by the general formula [6], 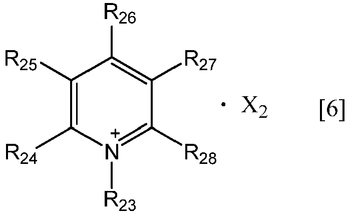 (wherein R₂₃ represents an alkyl group having 1 to 6 carbon atoms; R₂₄ to R₂₈ each independently represent hydrogen atom, or an alkyl group having 1 to 6 carbon atoms; and X₂ represents a counter anion.),
the compound represented by the general formula [7], 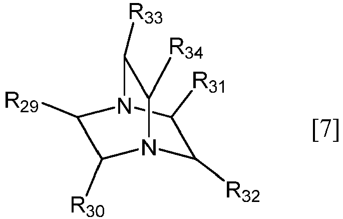 (wherein R₂₉ to R₃₄ each independently represent hydrogen atom, or an alkyl group having 1 to 6 carbon atoms), and
the compound represented by the general formula [8], 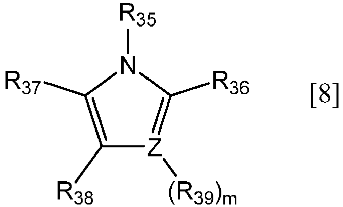 (wherein Z represents nitrogen atom or carbon atom; m represents an integer of 0 or 1, provided that m represents 0 when Z is nitrogen atom, and m represents 1 when Z is carbon atom; R₃₅ to R₃₉ each independently represent hydrogen atom, or an alkyl group having 1 to 6 carbon atoms.).

The genomic DNA in the bisulfite reaction may be provided as a solution dissolving the genomic DNA, and the solution includes the one having, for example, a pH of 6 to 8, and dissolving it in a Good's buffer solution, such as MES, HEPES, a phosphate buffer solution, a TRIS buffer solution, a glycin buffer solution, a borate buffer solution, a sodium hydrogen carbonate buffer solution, sterilized water, or the like, and among them, the one dissolving it in sterilized water is preferable. DNA content in the solution is not especially limited, and is usually 10 to 100 ng in 1 to 10 µL of the solution.

Single strand formation treatment of the genomic DNA in the bisulfide reaction may be carried out according to single strand formation treatment known per se. It includes thermal treatment which is carried out, for example, by heating at usually 80 to 100 °C, and preferably 85 to 95 °C, for usually 30 seconds to 10 minutes, and preferably 1 to 3 minutes, or alkali treatment which is carried out, for example, by making DNA contacted with an alkali, or the like, and heat treatment is preferable. Particularly, when the bisulfite reaction is carried out in the presence of the compounds shown by the general formulae [1] to [8], the reaction can be proceeded without decomposition of DNA, even reaction temperature is raised to 80 to 100 °C. Therefore, the genomic DNA may be subjected to the bisulfite reaction at 80 to 100 °C. Single strand formation treatment and the bisulfite reaction may be proceeded simultaneously, by which treatment is simplified, and therefore, this method is more preferable in carrying out single strand formation treatment.

The alkali treatment is specifically carried out, for example, by adjusting the solution to an alkaline state, having usually a pH of 10 to 14, and preferably a pH of 12 to 14, by the addition of an alkali or an aqueous solution thereof, to the genomic DNA, or a solution containing the genomic DNA. The alkali includes an alkali metal hydroxide, for example, sodium hydroxide, potassium hydroxide, or the like; an alkaline earth metal hydroxide, for example, barium hydroxide, magnesium hydroxide, calcium hydroxide, or the like; an alkali metal carbonate, for example, sodium carbonate, or the like; ammonia, amines, and among them, the alkali metal hydroxide, for example, sodium hydroxide, potassium hydroxide, or the like, is preferable, and further among them, sodium hydroxide is particularly preferable.

In the bisulfite reaction, the sulfite in the reaction of DNA and the sulfite includes, for example, sodium hydrogen sulfite, ammonium sulfite, or the like, and sodium hydrogen sulfite is preferable. Use amount thereof is usually added, so that final concentration in the reaction solution attains 1 to 6 mol / L, relative to 1 to 500 µL of the solution containing 50 to 500 ng of DNA. The reaction of the DNA and the sulfite is carried out usually at 30 to 100 °C, preferably 50 to 100 °C, and more preferably 80 to 95 °C, for usually 60 minutes to 20 hours, preferably 60 minutes to 5 hours, and more preferably 60 minutes to 120 minutes. Cytosine is sulfonated by the reaction.

Hydrolysis of thus sulfonated cytosine in the bisulfite reaction is not especially limited, as long as it is a method usually carried out in this field, and it may be carried out by heating at usually 30 to 100 °C, and preferably 80 to 95 °C, for usually 60 minutes to 20 hours, preferably 60 minutes to 5 hours, and more preferably 60 minutes to 120 minutes. It should be noted that the hydrolysis treatment may be carried out simultaneously with the reaction of DNA and the sulfite. In that case, reaction temperature and reaction time in the reaction of DNA and the sulfite may be set in accordance with hydrolysis condition of sulfonated cytosine.

The hydrolyzed DNA is preferably subjected to purification treatment before carrying out desulfonation treatment. The purification treatment is carried out for the purpose of removing high concentration sulfite, or the like, to be used in the bisulfite reaction, and may be carried out according to a purification method for DNA usually carried out in this field. Specifically, for example, it includes a method in which a chaotropic agent, such as guanidine hydrochloride, sodium iodide, or the like, is added to DNA or a solution containing DNA, and it is separated and purified by an HPLC method, or the like; or for example, extraction and purification using a mixed solution of phenol/ chloroform/ isoamyl alcohol; an alcohol precipitation method; purification by a column packed with silica gel; a filter filtration method, or the like, and among them, the alcohol precipitation method is preferable. The alcohol precipitation method is specifically carried out as follows.

That is, to 10 µL of a solution containing DNA after hydrolysis treatment, usually 40 to 110 µL of alcohol, and 30 to 100 µL of a buffer solution are added, and then centrifugal separation is carried out. After centrifugal separation, DNA can be separated and purified by removing the supernatant, and washing it with alcohol. When the alcohol and the buffer solution are added, 0.1 to 1 µL of ethachinmate or glycogen may be added to 10 µL of the solution containing DNA, to make removal of the supernatant easy after separation. The alcohol includes ethanol, isopropanol, butanol, or the like, and isopropanol is particularly preferable. When isopropanol is used, only DNA can be efficiently precipitated, and therefore, the reaction can be efficiently promoted. The buffer solution includes, for example, a Good's buffer solution, such as MES, HEPES, or the like; a phosphate buffer solution; a TRIS buffer solution; a glycin buffer solution; a borate buffer solution; a sodium hydrogen carbonate buffer solution, or the like, and among them, the Good's buffer solution, such as MES, HEPES, or the like, the TRIS buffer solution, or the like, is preferable, and the TRIS buffer solution is particularly preferable. pH of these buffer solutions is usually 7 to 8, and preferably 7 to 7.5, and buffer agent concentration in the buffer solution is usually in a range of 0.1 to 5 mol/ L, and preferably 0.1 to 2 mol/ L. The centrifugal separation is not especially limited, as long as it is an aspect usually carried out in this field, and is usually carried out under 12,000 to 22,000 G for 10 to 30 minutes.

The desulfonation reaction, in the presence of an alkali in the bisulfite reaction, includes the same method as alkali treatment in the section of the single strand formation treatment, and a preferable aspect thereof also includes the same one. Specifically, for example, it may be carried out as follows.

That is, to 10 µL of the solution after hydrolysis treatment, or the solution having been subjected to purification treatment after hydrolysis treatment, usually, 1 to 10 µL, preferably 1 to 5 µL of a 0.5 to 3 mol/ L alkali aqueous solution is added to adjust pH of the reaction solution to 11 to 14, and preferably 12 to 14, and the desulfonation reaction is carried out by warming for usually 5 to 60 minutes, and preferably 5 to 30 minutes, at usually 25 to 70°C, and preferably 30 to 50°C.

Explanation will be given below on a preferable specific example of the bisulfite reaction.

That is, for example, DNA is extracted from target cells using a DNA extraction kit, or the like, and then 1 µg of DNA is dissolved, for example, in 5 to 15 µL of sterilized water. To 3 to 5 µL of the solution, for example, 50 to 100 µL of 2 to 5 mol/ L sodium hydrogen sulfite solution (pH: 5.0 to 7.0), and the compounds shown by the general formulae [1] to [8], or 1 to 12 µL of an aqueous solution containing them are added, so that concentration in a reaction solution attains 3 to 10%, when the compounds shown by the general formulae [1] to [8] are liquids, and concentration in the reaction solution attains 1 to 1000 mmol / L, when the compounds represented by the general formulae [1] to [8] are solids, and then heated at 80 to 100°C, for 60 to 120 minutes. By the reaction, the genomic DNA becomes single-stranded DNA, cytosine in the single-stranded DNA is sulfonated, and simultaneously, sulfonated cytosine can be hydrolyzed. Subsequently, the 1 mol/ L TRIS buffer solution (pH: 7.0 to 8.0), and isopropanol are added each in 5 to 10 times amount of the solution after hydrolysis, in a ratio of 40: 60 to 60: 40, and preferably 40: 60 to 50: 50, to precipitate DNA after hydrolysis. In this case, when 1 to 3 µL of ethachinmate or glycogen is added, confirmation of DNA precipitation becomes easy. Then, centrifugal separation is carried out under 12,000 to 20,000 G for 10 to 20 minutes to remove the supernatant, and the resulting DNA is washed with ethanol. By this treatment, DNA after hydrolysis can be extracted and purified. Further, 1 µg of the resulting DNA is dissolved in, for example, 30 to 40 µL of sterilized water, and 5 to 20 µL of 1 to 3 mol/ L sodium hydroxide solution is added to the solution, and the solution is subjected to a reaction at 30 to 40 °C, for 20 to 60 minutes for desulfonation. Then, if necessary, purification is carried out to remove low molecular weight DNA, by using, for example, a commercially available kit, or the like. By this treatment, the bisulfite reaction relevant to the present invention is completed, and DNA, where non-methylated cytosine in the genomic DNA is efficiently converted to uracil (hereinafter it may be abbreviated as uracilated DNA), is obtained.

### [Acquisition of DNA having the target base sequence relevant to the present invention]

The acquisition method for DNA, having the objective nucleotide sequence relevant to the present invention (nucleotide sequence comprising a part of or all of the nucleotide sequence shown in sequence number 1), from the genomic DNA which has been subjected to the bisulfite reaction, is carried out, by subjecting the uracilated DNA to the PCR reaction, using a primer designed to amplify DNA having the target base sequence relevant to the present invention. According to this method, it is possible to obtain a DNA amplified substance having the objective nucleotide sequence, in which non-methylated cytosine is converted to uracil.

The PCR reaction may be carried out according to a known method per se, for example, the method described in Nucleic Acids Research, 1991, Vol. 19, 3749, and Bio Techniques, 1994, Vol. 16, 1134-1137, and is specifically carried out as follows. That is, to 1 to 100 ng of nucleic acid amount of the uracilated DNA as a template, by the addition of 2 kinds of primers each usually in 0.1 to 100 pmol, and preferably 0.1 to 50 pmol, and DNA polymerase usually in 1 to 10 units, preferably 2.5 to 5 units, as well as 4 kinds of mixed deoxyribonucleotide triphosphates (dNTPs) usually in 0.01 to 20 µmol, and preferably 0.01 to 10 µmol, and in the buffer solution, for example, a tricine buffer solution, a TRIS hydrochloride buffer solution, or the like, having a pH of 7 to 9, by carrying out 20 to 40 cycles, by setting as 1 cycle, for example, (1) 93 to 98 °C, 1 to 10 minutes → (2) 93 to 98 °C, 10 to 30 seconds → (3) 50 to 60 °C, 10 to 30 seconds → (4) 68 to 72 °C, 30 seconds to 5 minutes, the uracilated DNA can be amplified. It should be noted that heating may be carried out at 68 to 72 °C, for 1 to 5 minutes, for 3'-adenine addition, after carrying out the cycle, in response to DNA polymerase to be used.

In the PCR reaction, it is preferable that the resulting DNA is purified after the reaction, by a purification method usually carried out in this field, for example, by a method for extraction with a mixed solvent of phenol/chloroform/isoamyl alcohol, alcohol precipitation, column purification, filter filtration, or the like. In addition, it is more preferable that DNA having the target base pair (bp) is extracted after the purification. The extraction method includes a known method per se, for example, a method using agarose gel electrophoresis, a method using liquid chromatography, a method using electrophoresis on polyacrylamide gel described in an enlarged edition of Labo-manual genetic engineering, 1990, 27-28, or the like. In addition, DNA obtained by the PCR reaction (PCR product) may be subjected further to the PCR reaction under similar conditions to yield more quantity.

Two kinds of primers in the PCR reaction may be those designed to amplify DNA having the objective nucleotide sequence relevant to the present invention. Number of the nucleotides are usually 20 to 45, preferably 22 to 40, and more preferably 25 to 35. Specific examples of the two kinds of the primers include, for example, the following ones.

Forward primer:
   GGTTTGGTTAAGTGTTTGGTTAGGGG (SEQ ID NO: 4)
   GTAGTTAGTTTTAGTATTTAGTTTTTGG (SEQ ID NO: 5)
Reverse primer:
   CCTAACTACCCCCACTAATTCCCACTC (SEQ ID NO: 6)
   CCCTCTCTACTTCTCTCTCCTACCTTCTC (SEQ ID NO: 7)

The DNA polymerase in the PCR reaction may be any DNA polymerase, as long as it is usually used in this field, and, the one having 5'→3' polymerase activity is preferable, and among them, the one having exonuclease activity but does not have 3'→5' exonuclease activity is more preferable. Specifically, for example, a mutant type Taq DNA polymerase, such as KAPA2G polymerase, Taq DNA polymerase, or Tth DNA polymerase, is preferable, and among them the KAPA2G polymerase is particularly preferable.

The dNTPs in the PCR reaction are not especially limited, as long as they are a mixture of four kinds of deoxyribonucleotide triphosphates (dATP, dCTP, dGTP, dTTP), usually used in this field.

Explanation will be given below on specific preferable examples of the method for obtaining DNA having the objective nucleotide sequence pertaining to the present invention.

That is, firstly, as described in the section of [Bisulfite reaction], the genomic DNA is subjected to single strand formation treatment, and then subjected to the bisulfite reaction to obtain uracilated DNA. Thereafter, the resulting uracilated DNA was subjected to the PCR reaction, using primers designed to amplify the DNA having the objective nucleotide sequence pertaining to the present invention. Specifically, to 1 to 3 µL of a solution containing 1 to 100 ng of the uracilated DNA obtained by the bisulfite reaction, 5 to 10 µL of a 1 to 10 µmol/L forward primer of the amplification target DNA (for example, GTAGTTAGTTTTAGTATTTAGTTTTTGG: SEQ ID NO: 5) and 5 to 10 µL of a 1 to 10 µmol/L reverse primer of the amplification target DNA (for example, CCCTCTCTACTTCTCTCTCCTACCTTCTC: SEQ ID NO: 7), 5 to 10 µL of a 1 to 5 mmol/L mixed solution of 4 kinds of deoxyribonucleotide triphosphates (dNTPs), as well as 10 to 20 µL of 1 to 5 units KAPA DNA polymerase are added, and for example, by setting a reaction at 93 to 98°C, for 1 to 10 minutes → at 93 to 98°C, for 10 to 30 seconds → at 50 to 60°C, for 10 to 30 seconds → at 68 to 72°C, 68 to 72°C, for 30 seconds to 5 minutes, as one cycle, 20 to 40 cycles of the reaction are carried out. Thereby, the uracilated DNA is amplified, and the DNA having the objective nucleotide sequence pertaining to the present invention, in which unmethylated cytosine has been converted into uracil (hereinafter, it may sometimes be referred to as the DNA having the uracilated objective nucleotide sequence) can be obtained.

It should be noted that, when adenine-adding activity of the 3'-terminal of DNA polymerase is utilized for the purpose of employing a TA cloning method, as a method for incorporating the DNA having the uracilated objective nucleotide sequence into a vector, to be described later, a further reaction at 68 to 72°C for 30 seconds to 5 minutes may be carried out, after completion of the reaction cycles. Next, the amplified DNA is subjected to electrophoresis using, for example, agarose gel or non-denaturing polyacrylamide gel, and the DNA having desired chain length is extracted. It should be noted that, if needed, the resulting DNA may be purified, for example, by extraction with a mixed solution of phenol/chloroform/isoamyl alcohol, or the like.

### Measurement of methylation ratio of cytosine of the -CG- sequence, and determination method for canceration

The measurement of methylation ratio of cytosine of the -CG-sequence is carried out, by subjecting the DNA having the uracilated objective nucleotide sequence to nucleotide sequence analysis by a sequencer or the like, comparing the resulting nucleotide sequence with the normal nucleotide sequence, measuring number of methylated cytosine of the -CG- sequence of the objective nucleotide sequence pertaining to the present invention, or measuring number of methylation of cytosine of the specific -CG- sequence in the objective nucleotide sequence pertaining to the present invention, and calculating methylation ratio, based on the values. In the determination method for canceration, when methylation ratio thereof is 30% or more, preferably 40% or more, and more preferably 50% or more, it is determined that cells are cancerated.
When the objective nucleotide sequence pertaining to the present invention is the nucleotide sequence represented by SEQ ID NO: 2, or the nucleotide sequence represented by SEQ ID NO: 3, or when methylation of cytosines of the -CG- sequences of the circle 8 to circle 17 or circle 13 to circle 17 in Fig.7 is measured, and at a methylation ratio of 40% or more, preferably 50% or more, and more preferably 60% or more, it can be determined that cells are cancerous, and determination of cancer can be carried out in better precision.

The nucleotide sequence analysis is not especially limited, as long as it is a method for analyzing nucleotide sequence usually carried out in this field, and for example, it may be carried out according to a usual method, such as a fluorescent dye terminator method, the Sanger's method, described in Lab Manual for Genetic Engineering, and Handbook of Gene Technology, or the like. Specifically, for example, it may be carried out by incorporating the DNA having the uracilated objective nucleotide sequence in a vector, and transfecting the resulting recombinant vector into competent cells, culturing the competent cells, and extracting a plasmid containing the DNA having the uracilated objective nucleotide sequence therefrom, and decoding, for example, by a sequencer, or the like, using the plasmid.

The method for incorporating the DNA having the uracilated objective nucleotide sequence into a vector includes, specifically, for example, a method for inserting the DNA having the uracilated objective nucleotide sequence into a vector, using T4 DNA ligase, after the vector, such as plasmid, cosmid, and phagemid, and the DNA having the uracilated objective nucleotide sequence, are blunt-ended by T4 DNA polymerase, or the like, or a TA cloning method, in which adenine (A) is added to the DNA having the uracilated objective nucleotide sequence, and the adenine-added DNA having the uracilated objective nucleotide sequence is incorporated into a thymine base-added vector, using T4 DNA ligase. Among them, the TA cloning method is preferable because it does not require cleaving of both the DNA to be inserted and the vector, by a restriction enzyme, and thus it is simple.

The TA cloning method is carried out, specifically, for example, as follows. That is, 1 to 5 units of Taq DNA polymerase are added, relative to 100 ng of the DNA having the uracilated objective nucleotide sequence after the PCR reaction, and by subjecting it to a reaction at 55 to 75°C for 10 to 30 minutes, adenine is added to the 3'-terminal of the DNA having the uracilated objective nucleotide sequence. It should be noted that, in the PCR reaction, when the DNA polymerase having adenine-adding activity of the 3'-terminal is used, the adenine addition step is not necessary. In addition, in the reaction, 0.01 to 20 nmol of dATP, relative to 100 ng of the complementary DNA of the uracilated DNA, may be added to the reaction solution, however, by adding Taq DNA polymerase to the PCR reaction solution, this addition step can be skipped. In addition, in the reaction, 0.01 to 20 nmol of dATP, relative to 100 ng of the DNA having the uracilated objective nucleotide sequence, may be added to the reaction solution. When the PCR reaction solution is used directly for the TA cloning, the addition of dATP is not necessary because of remaining dATP. In addition, with respect to the adenine-added DNA having the uracilated objective nucleotide sequence, it is preferable to purify the resulting DNA after the synthesis reaction, by a method, such as extraction with a mixed solution of phenol/chloroform/isoamyl alcohol, alcohol precipitation, column purification, filtration by a filter, or the like. Subsequently, a recombinant vector, in which the DNA having the uracilated objective nucleotide sequence is incorporated, can be obtained by the addition of a thymine base-added vector for E. coli transformation and 300 to 3000 units of T4 DNA ligase, relative to 10 to 100 ng of the adenine-added DNA having the uracilated objective nucleotide sequence, and by subjecting it to a reaction at 10 to 40°C for 30 to 90 minutes.

A method for transforming the recombinant vector into the competent cells includes, for example, a heat shock method for heating at 35 to 45°C for 20 to 90 seconds, or an electroporation method in which an electric pulse of 1.5 to 2.5 kV is applied, or the like. As the competent cells to be used herein, anyone can be used, as long as it is usually used E. coli or B. subtilis or the like, and its use amount may be set as appropriate within a range usually used.

Cultivation of the competent cells is carried out, for example, on a medium, such as an LB agar medium containing 30 to 150 µg/mL of ampicillin, or an M9 agar medium, or the like, containing 30 to 150 µg/mL of ampicillin, at 30 to 40°C for 12 to 20 hours. It should be noted that, as the medium, either one of a natural medium or a synthetic medium, or the like, may be employed, as long as it contains a carbon source, a nitrogen source and inorganic salts which are nutritional sources of bacteria, and yeast extract as a growth factor, and it enables to culture the transformant efficiently. The carbon source includes carbohydrates, such as glucose, fructose, sucrose and starch; organic acids, such as acetic acid and propionic acid; and alcohols such as ethanol and propanol. The nitrogen source includes ammonia, an ammonium salt of an inorganic acid or an organic acid, such as ammonium chloride, ammonium sulfate, ammonium acetate, and ammonium phosphate; or other nitrogen-containing compounds, as well as, peptone, tryptone, meat extract, Corn Steep Liquor, or the like. The inorganic salts include monobasic potassium phosphate, dibasic potassium phosphate, magnesium phosphate, magnesium sulfate, sodium chloride, ferrous sulfate, manganese sulfate, copper sulfate, calcium carbonate, and the like.

As an extraction method for the plasmid containing the DNA having the uracilated objective nucleotide sequence, from the cultured competent cells, for example, firstly, the DNA derived from the plasmid in a colony, which has the DNA having the uracilated objective nucleotide sequence, is amplified by a colony PCR method. After that, the method may be carried out by confirming whether the objective plasmid is amplified in the colony, for example, by an electrophoresis method, and by extraction of the objective plasmid from the colony, where insertion of the objective plasmid was confirmed.

The colony PCR method is carried out, for example, as follows. That is, to the cultured colony, each usually 0.1 to 100 pmol, and preferably 0.1 to 50 pmol of 2 kinds of PCR primers for detection of the objective nucleotide sequence pertaining to the present invention, usually 0.01 to 20 nmol, and preferably 0.01 to 10 nmol of 4 kinds of mixed deoxyribonucleotide triphosphates (dNTPs), and usually 1 to 10 units, and preferably 1 to 5 units of DNA polymerase are added. Then, in a buffer solution, such as a Tricine buffer solution, a Tris hydrochloric acid buffer solution, or the like, for example, by setting a reaction (1) at 93 to 98°C, for 1 to 10 minutes → (2) at 93 to 98°C, for 10 to 30 seconds → (3) at 50 to 60°C, for 10 to 30 seconds → (4) at 68 to 72°C, for 30 seconds to 5 minutes, as 1 cycle, 25 to 40 cycles of the reaction are carried out. Two kinds of the primers include the one which is designed to be capable to amplifying the objective DNA, that is, the one containing all of or a portion of the DNA having the uracilated objective nucleotide sequence, or the sequence derived from the vector which locate at both ends of the inserted DNA having the uracilated objective nucleotide sequence.The sequences derived from the vector, which locate in the both end of the DNA having the uracilated objective nucleotide sequence, are preferable. That is, in the DNA amplification method of the present invention, and in methylated cytosine detection method of the present invention, since unmethylated cytosine is uracilated, and the uracil is read as thymine at the time of the PCR reaction, the DNA having the uracilated objective nucleotide sequence result in to be composed of 3 nucleotides when all cytosines are unmethylated cytosines. Therefore, in order to carry out the PCR reaction efficiently, the sequence derived from the vector, located at the both ends of the DNA having the uracilated objective nucleotide sequence, which can be composed of 4 nucleotides, is preferable. Number of nucleotides of the primer is usually 12 to 30, preferably 15 to 25, and more preferably 18 to 22. The DNA polymerase may be any DNA polymerase, as long as it is usually used in this field, and includes specifically, for example, a Taq DNA polymerase, a Tth DNA polymerase, and a KOD DNA polymerase, or the like, and among them, the Taq DNA polymerase, the KOD DNA polymerase, or the like is preferable.

The electrophoresis method, to be carried out after the colony PCR reaction, may be any electrophoresis method, usually used in this field, as long as it is capable of determining number of nucleotides based on mobility. Thus, an agarose gel electrophoresis method is preferable. It should be noted that, the electrophoretic condition in the electrophoresis method may be set as appropriate according to a well-known method.

As a method for taking out a plasmid from the colony part, the plasmid may be extracted by a well-known plasmid extraction method, for example, an alkaline SDS method after shaking culture, described in Lab Manual for Genetic Engineering (Maruzen Co., Ltd.), and Handbook of Gene Technology (Yodosha Co., Ltd.). or the like. It should be noted that, the extraction of the plasmid may be carried out using a commercially available kit. For the culture medium in the shaking culture, the same medium as described in the section of culture of the competent cells can be used, except for preparing a solution without using agarose, and preferable culture time and culture temperature are also the same range as described in the section of the competent cells.

Methylated cytosine can be detected by comparing the nucleotide sequence subjected to the bisulfite reaction, obtained by the nucleotide sequence analysis, and the nucleotide sequence of normal DNA in which cytosine is not methylated. That is, in the bisulfite reaction pertaining to the present invention, since all cytosines other than methylated cytosine (unmethylated cytosine) are uracilated, by finding out cytosine not uracilated in the resulting nucleotide sequence, methylated cytosine can be detected. Methylation ratio is calculated by dividing number of methylated cytosines in the -CG- sequence of the objective nucleotide sequence pertaining to the present invention, by number of cytosines of the -CG- sequence. When methylation ratio thereof is 30% or more, preferably 40% or more, and more preferably 50% or more, cells are determined to be cancerous. In addition, in the case where the objective nucleotide sequence pertaining to the present invention is the nucleotide sequence represented by SEQ ID NO: 2, or the nucleotide sequence represented by SEQ ID NO: 3, as well as, in the case where methylation of cytosines of the -CG- sequences of circle 8 to circle 17 or circle 13 to circle 17 in Fig. 7 is measured, when methylation ratio is 40%, preferably 50%, and more preferably 60% or more, cells are determined to be cancerous, and determination of cancer can be made in better precision.

Explanation will be given below on preferable examples of the determination method for canceration of the present invention.

That is, firstly, as described in the sections of "Bisulfite reaction" and "Acquisition of DNA having the objective nucleotide sequence pertaining to the present invention", the genomic DNA is subjected to single strand formation treatment, then subjected to the bisulfite reaction and the PCR reaction in this order, and the DNA having the uracilated objective nucleotide sequence is obtained. To 1 to 5 µL of sterile water, or the like, containing 10 to 100 ng of the DNA having the uracilated objective nucleotide sequence, 1 to 3 µL of the 10 to 100 ng thymine-added vector for E. coli transformation, and 1 to 3 µL of 300 to 3000 units of T4 DNA ligase are added and subjected to a reaction at 10 to 20°C for 30 to 240 minutes to obtain a recombinant vector incorporated with the DNA having the uracilated objective nucleotide sequence. It should be noted that, in the PCR reaction, when a DNA polymerase having no adenine-adding activity, for example, α-DNA polymerase is used, adenine is added to the DNA having the uracilated objective nucleotide sequence, prior to incorporation into the vector. The adenine adding method may be carried out, for example, by the addition of 0.5 to 1 µL of 1 to 5 units Taq DNA polymerase, to 5 to 10 µL of the PCR reaction solution containing 100 ng to 1 µg of the DNA having the objective nucleotide sequence pertaining to the present invention, and subjecting it to a reaction at 55 to 75°C for 10 to 30 minutes, and thus adding adenine to the 3'-terminal of the DNA having the objective nucleotide sequence pertaining to the present invention. It should be noted that, after the adenine reaction, it is desirable to subject it to purification operation.

After acquisition of the recombinant vector, 10 to 100 ng of the resulting recombinant vector is added to 10⁸ to 10⁹ cells of the competent cells, and transformation is carried out by heating at 35 to 45°C for 20 to 90 seconds. Furthermore, the cells are cultured on an agar medium containing, for example, 30 to 150 µg/mL of ampicillin, 1% (w/v) of tryptone, 0.5% (w/v) of yeast extract, and 1% (w/v) of sodium chloride, at 30 to 40°C for 12 to 20 hours. Subsequently, the resulting culture is subjected to the colony PCR. Specifically, to 1 to 10 µL of colony-dissolved sterile water, each 1 to 5 µL of 2 kinds of 1 to 10 µmol/L of PCR primers, which are designed to be capable of amplifying the objective DNA, usually 1 to 5 µL of 1 to 5 mmol/L of 4 kinds of mixed deoxyribonucleotide triphosphates (dNTPs), as well as 0.5 to 1 µL of 1 to 5 units Taq DNA polymerase are added.Then, in a buffer solution such as a Tricine buffer solution or a Tris hydrochloric acid buffer solution, at pH 7 to 9, by setting a reaction (1) at 93 to 98°C, for 1 to 10 minutes → (2) at 93 to 98°C, for 10 to 30 seconds → (3) at 50 to 60°C, for 10 to 30 seconds → (4) at 68 to 72°C, for 30 seconds to 5 minutes, as 1 cycle, 25 to 40 cycles of the reaction are carried out. After that, presence of the objective DNA in the colony is determined, by an agarose gel electrophoresis method, and confirmed colonies are collected. After carrying out shaking culture of the collected colonies in an LB medium, the objective DNA is taken out from the culture fluid using, for example, a commercially available plasmid extraction kit, or the like, and the nucleotide sequence of the DNA is analyzed by a sequencer, or the like. By comparing the resulting nucleotide sequence with the normal nucleotide sequence, in which cytosine is not methylated, methylation ratio of cytosine of the -CG- sequence in the objective nucleotide sequence pertaining to the present invention is measured. From methylation ratio thereof, canceration ratio of cells can be determined.

Explanation will be given below in more detail on the present invention by referring to Examples, and the like, however, the present invention should not be limited thereto in any way.

### EXAMPLES

### Example 1: Methylation analysis of the FOXB2 gene in normal cells

### (1) Extraction of the genomic DNA

Using the following five kinds of cells, the genomic DNA was extracted by QuickGene SP kit DNA tissue (produced by Kurabo Industries Ltd.), according to the attached instruction manual.
- Normal human skin fibroblast cells (hiPS, Cell name: 201B7)
- Normal human skin fibroblast cells (HDF)
- Normal human lung fibroblast cells (WI-38)
- Normal human lung diploid cells (CCD-8Lu)
- Normal human fetal cells (HE23)

### (2) Bisulfite reaction

To each 400 ng of 5 kinds of the genomic DNA obtained in (1), and Human Genomic DNA (produced by Promega Corporation), as the genomic DNA from normal human whole blood, distilled water was added to prepare 10 µL of an aqueous solution. Then, each of them was subjected to the bisulfite reaction, using an EpiSight Bisulfite Conversion Kit (produced by Wako Pure Chemical Industries, Ltd.), according to the instruction manual.

### (3) Amplification of a FOXB2 promoter region (the PCR reaction)

To each 1 µL of the bisulfite reaction products obtained in (2), 17.3 µL of distilled water, 2.5 µL of 10 x Buffer for Blend Taq (produced by Toyobo Co., Ltd.), 2 µL of 2 mM dNTPs (produced by Toyobo Co., Ltd.), 0.2 µL (0.5 U) of Blend Taq-Plus (produced by Toyobo Co., Ltd.), 1 µL (10 µM) of a forward primer, and 1 µL (10 µM) of a reverse primer were added and mixed, and used them as reaction solutions.

Nucleotide sequences of each primer are as follows. Amplification chain length generated by these primers is 493 bp.
Forward primer: GGTTTGGTTAAGTGTTTGGTTAGGGG (SEQ ID NO: 4)
Reverse primer: CCTAACTACCCCCACTAATTCCCACTC (SEQ ID NO: 6)

Each reaction solution was set to a thermal cycler, and a reaction was carried out under the following conditions.
at 94°C for 2 minutes,
by setting at 94°C for 20 seconds → at 55°C for 20 seconds → at 72°C for 30 seconds, as one cycle, for 36 cycles,
at 72°C for 2 minutes.

After that, to 25 µL of each PCR amplification product, 5 µL of a 6 x Loading Buffer Double Dye (produced by Nippon Gene Co., Ltd.) was added and mixed, and the mixture was subjected to electrophoresis on 1.5% agarose gel. After electrophoresis, gel was stained with a GelRed nucleic acid gel staining solution (produced by Wako Pure Chemical Industries, Ltd.), and the PCR amplification product was recovered, using a QIAquick Gel Extraction Kit (produced by Qiagen, Inc.), according to the attached instruction manual.

### (4) The PCR reaction for adding a restriction enzyme-recognition site to the PCR amplification product

To each 1 µL of the PCR amplification product obtained in (3), 17.3 µL of distilled water, 2.5 µL of 10 x Buffer for Blend Taq (produced by Toyobo Co., Ltd.), 2 µL of 2 mM dNTPs (produced by Toyobo Co., Ltd.), 0.2 µL (0.5 U) of Blend Taq-Plus (produced by Toyobo Co., Ltd.), 1 µL (10 µM) of the forward primer, and 1 µL(10 µM) of the reverse primer were added and mixed.

As the forward primer, the one having the following sequence, that is the PCR forward primer, to which the restriction enzyme-recognition site (Hind III: an underlined part in the sequence) was added, was used; and as the reverse primer, the one having the following sequence, that is the PCR forward primer to which the restriction enzyme recognition site (BamH I: underlined part in the sequence) was added, was used.
Forward primer:
   TTACCATAAGCTTGGTTTGGTTAAGTGTTTGGTTAGGGG (SEQ ID NO: 8)
Reverse primer:
   TAATTAAGGATCCCCTAACTACCCCCACTAATTCCCACTC (SEQ ID NO: 9)

Each of the reaction solutions was set to the thermal cycler, and the reaction was carried out under the following conditions at 94°C for 2 minutes, by setting at 94°C for 20 seconds → at 55°C for 20 seconds → at 72°C for 30 seconds as one cycle, for 36 cycles,
at 72°C for 2 minutes.

After that, to 25 µL of each PCR amplification product, 5 µL of the 6 x Loading Buffer Double Dye (produced by Nippon Gene Co., Ltd.) was added and mixed, and the mixture was subjected to electrophoresis on 1.5% agarose gel. After electrophoresis, the gel was stained with the GelRed nucleic acid gel staining solution (produced by Wako Pure Chemical Industries, Ltd.), and the PCR amplification product was recovered using the QIAquick Gel Extraction Kit (produced by Qiagen, Inc.), according to the attached instruction manual.

### (5) Restriction enzyme treatment

To 43 µL of each PCR amplification product obtained in (4), 5 µL of 10 x B Buffer (produced by Nippon Gene Co., Ltd.), 1 µL of Hind III (produced by Nippon Gene Co., Ltd.), and 1 µL of BamH I (produced by Nippon Gene Co., Ltd.) were added and mixed, then incubated at 37°C for 1 hour. Similarly, to 500 ng of pUC19 (produced by Nippon Gene Co., Ltd.), distilled water was added to make 43 µL, further, 1 µL of Hind III (produced by Nippon Gene Co., Ltd.), and 1 µL of BamH I (produced by Nippon Gene Co., Ltd.) were added and mixed, then incubated at 37°C for 1 hour. Next, 250 µL of a binding Buffer (5.5 M guanidine hydrochloride (produced by Wako Pure Chemical Industries, Ltd.), and 20 mM Tris-HCl, pH 6.6 (produced by Wako Pure Chemical Industries, Ltd.) was added and mixed, and then transferred it to an Econospin (manufactured by GeneDesign, Inc.) and centrifugally separated at 12000 x G, at room temperature for 1 minute to remove the solution in the tube. Subsequently, 500 µL of a washing Buffer (2 mM Tris-HCl, pH 7.5, produced by Nippon Gene Co., Ltd.), and 500 mL of 80% ethanol (produced by Wako Pure Chemical Industries, Ltd.) was added, and centrifugally separated at 12000 x G, at room temperature for 1 minute to remove the solution in the tube. Further, after additional centrifugal separation at 12000 x G, at room temperature for 1 minute, the tube was replaced with a new tube, and 25 µL of an elution Buffer (10 mM Tris-HCl, pH 8.5) (produced by Nippon Gene Co., Ltd.) was added, and centrifugally separated at 12000 x G, at room temperature for 1 minute. Thereby, the restriction enzyme-treated PCR amplification product and pUC19 were recovered.

### (6) Transformation

To 1 µL of each restriction enzyme-treated PCR amplification product, restriction enzyme-treated pUC19, and 2 µL of a DNA Ligation Kit (Mighty Mix) (produced by Takara Bio Inc.) were added and mixed, and incubated at 16°C for 1 hour. Next, the entire amount was transfected into 4 µL of ECOS™ Competent E. coli DH5α (produced by Nippon Gene Co., Ltd.), and spread on an ampicillin-added LB agar medium. After that, the colonies were cultured in the ampicillin-added LB medium at 37°C for 16 hours. Further, extraction of a plasmid was carried out using a Plasmid Kit SII (produced by Kurabo Industries Ltd.), according to the attached instruction manual.

Using the resulting plasmid, the nucleotide sequence was analyzed using contract sequencing service by Takara Bio Inc.

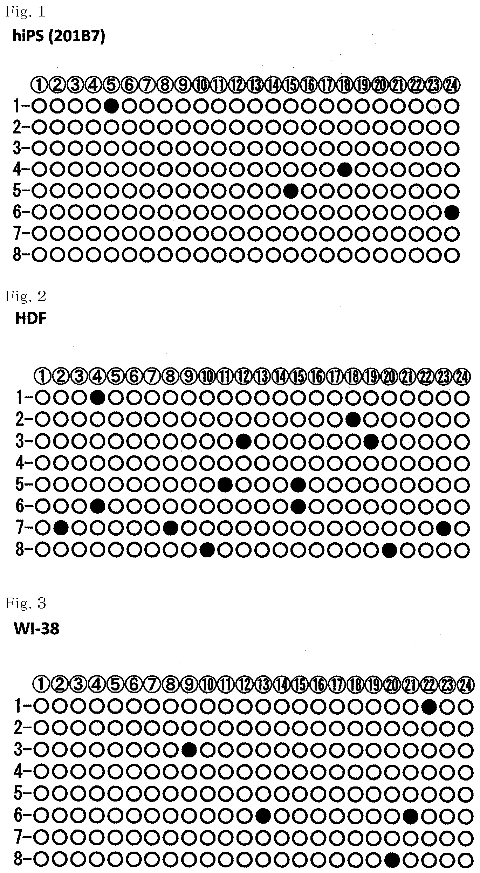

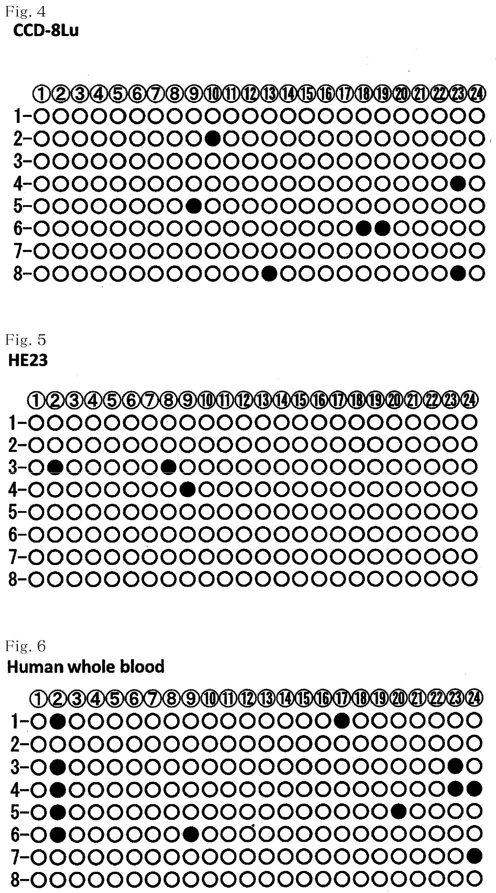

Based on the results of nucleotide sequencing, diagrams showing whether cytosine of CpG dinucleotide is unmethylated cytosine or methylated cytosine are shown in Fig. 1 to Fig. 6. It should be noted that, O in the diagrams indicates that cytosine of CpG dinucleotide is unmethylated, ● indicates that cytosine of CpG dinucleotide is methylated.

The relationship between the figure and cells or whole blood used was shown in the following Table 1.

**Table 1**

| Result | Cells or whole blood |
|---|---|
| Fig. 1 | Normal human skin fibroblast cells (hiPS) |
| Fig. 2 | Normal human skin fibroblast cells (HDF) |
| Fig. 3 | Normal human lung fibroblast cells (WI-38) |
| Fig. 4 | Normal human lung diploid cells (CCD-8Lu) |
| Fig. 5 | Normal human fetal cells (HE23) |
| Fig. 6 | Leucocyte (whole blood) |

In addition, positions of cytosines of the circled numbers in Figs. 1 to 6 were indicated in the nucleotide sequence of the FOXB2 promoter region described in Fig. 7 (the PCR amplification region, GenBank Accession No. AL353637: 106566-107058: SEQ ID NO: 10).

Methylation ratio of cytosines of CpG dinucleotides corresponding to the region of SEQ ID NO: 2 (circle 8 to circle 17 in the figure), and methylation ratio of cytosines of CpG dinucleotides corresponding to the region of SEQ ID NO: 1 (circle 6 to circle 21 in the figure), which were calculated based on the results of Fig. 1 to Fig. 6, are shown in Table 3 together with the results of Example 2.

### Example 2: Methylation analysis of the FOXB2 gene in cancer cells

Using 25 kinds of cancer cells in the following Table 2, the genomic DNA was extracted by QuickGene SP kit DNA tissue (produced by Kurabo Industries Ltd.), according to the attached instruction manual. Subsequent processing was carried out similarly as the method described in (2) to (6) in Example 1, and the nucleotide sequences of the FOXB2 gene of various cancer cells were analyzed.

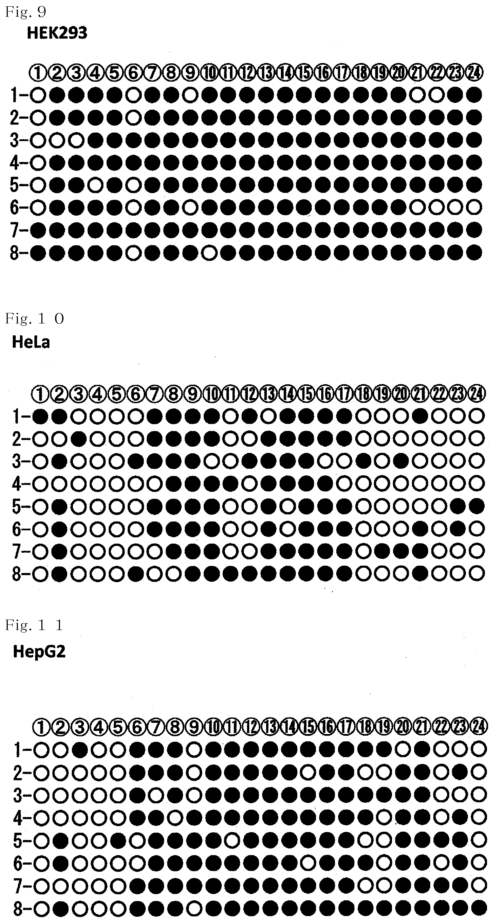

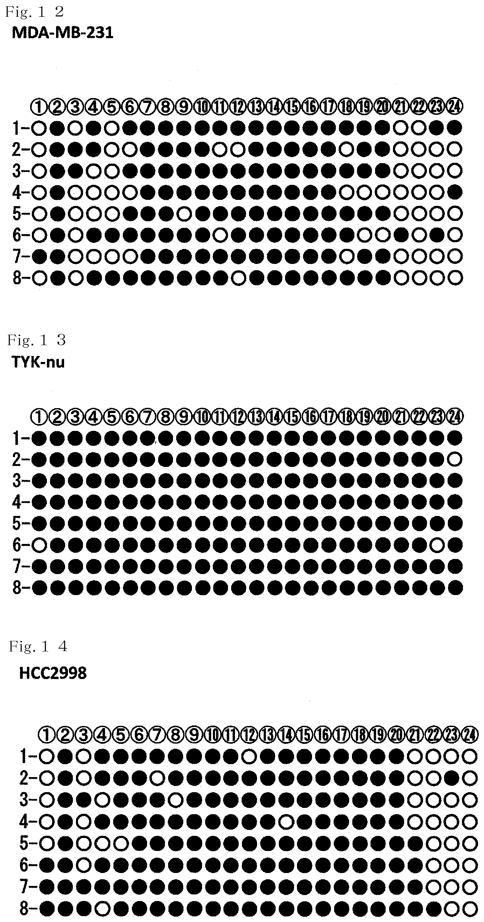

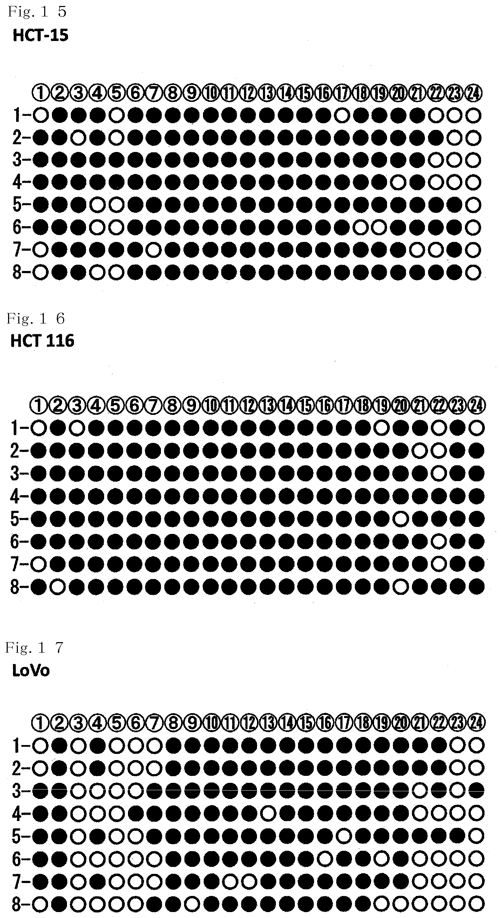

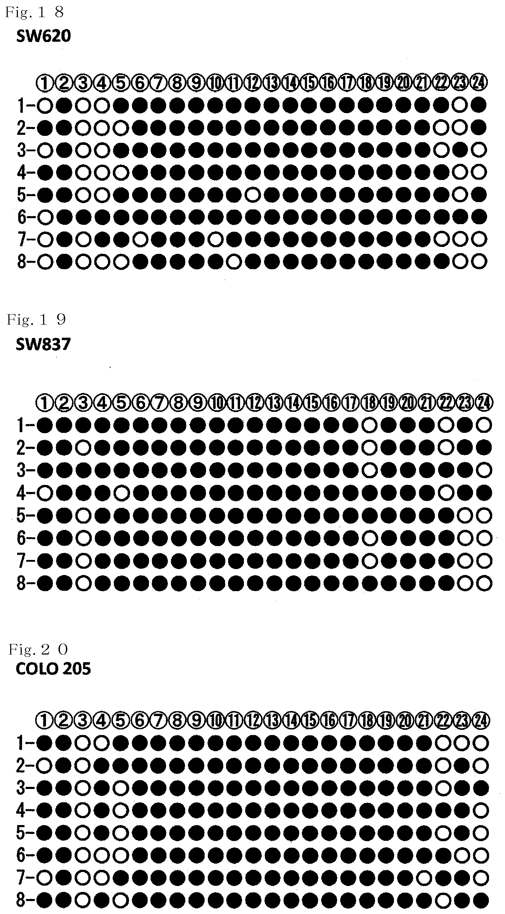

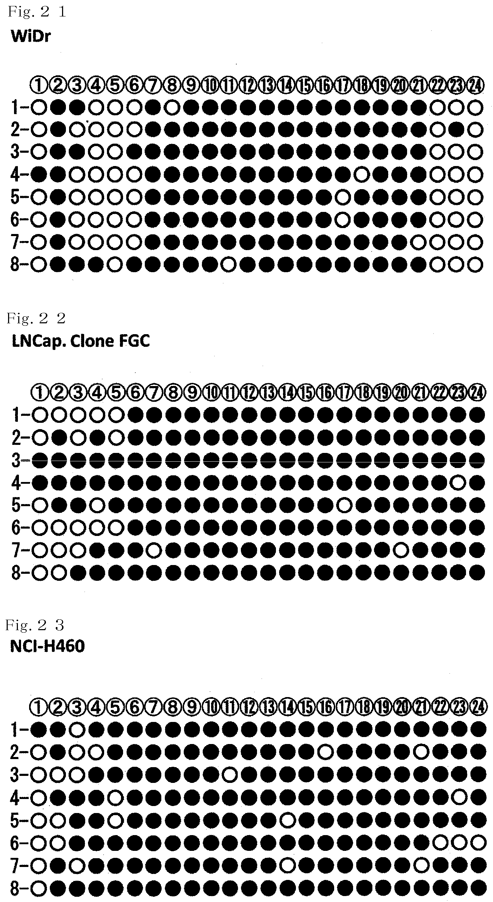

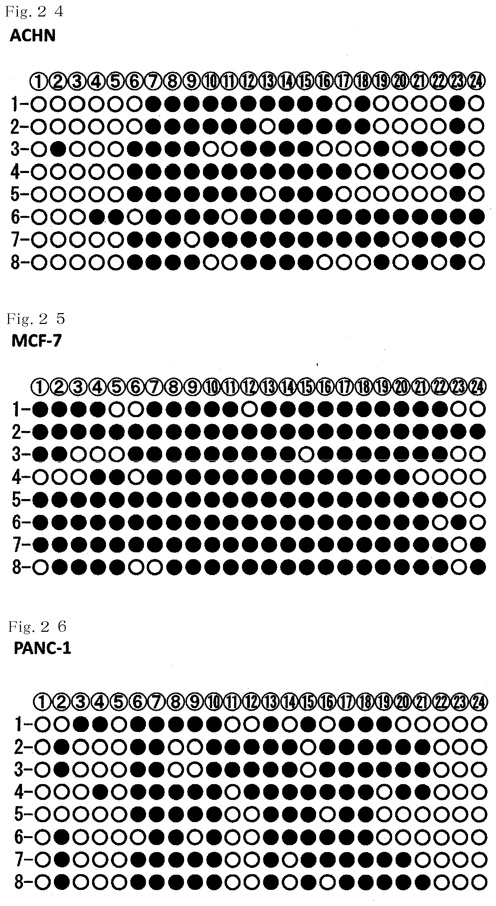

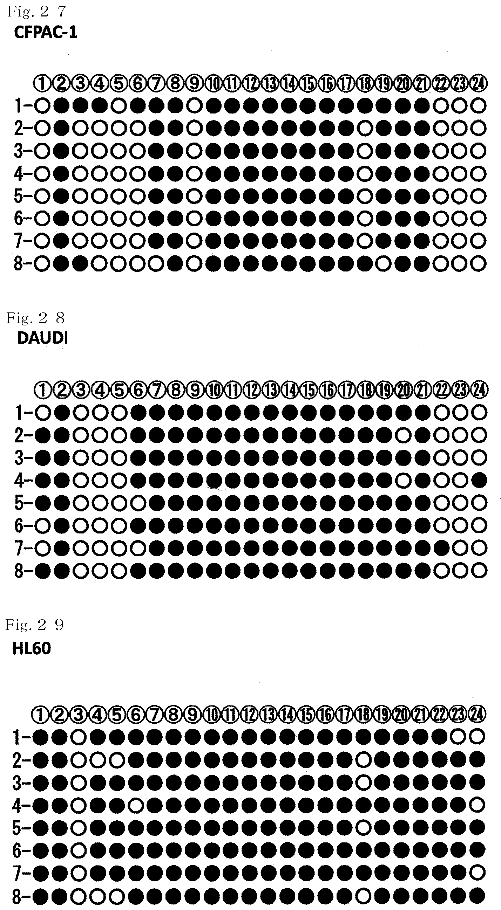

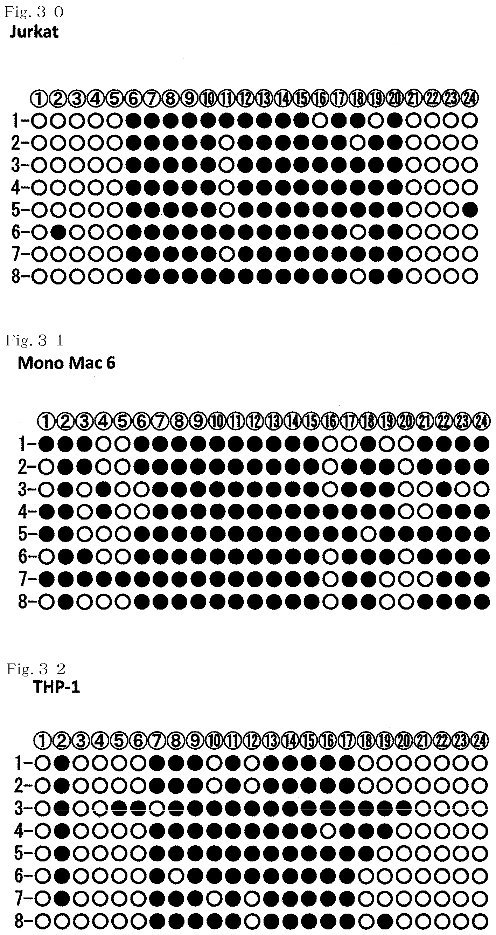

Based on the results of nucleotide sequencing, diagrams showing whether cytosine of CpG dinucleotides is unmethylated cytosine or methylated cytosine are shown in Fig. 8 to Fig. 32. It should be noted that, positions of cytosines of circled numbers in the figures are as described in Fig. 7. In addition, O in the diagram indicates that cytosine of CpG dinucleotide is unmethylated, ● indicates that cytosine of CpG dinucleotide is methylated.

The relationship between the figure and cells used was shown in the following Table 2.

**Table 2**

| Figure | Cells | Figure | Cells |
|---|---|---|---|
| Fig. 8 | IMR-32 (human abdominal neuroblastoma cells) | Fig. 21 | WiDr (human colon adenocarcinoma cells) |
| Fig. 9 | HEK293 (human embryonic kidney cells) | Fig. 22 | LNCap. Clone FGC (human prostate cancer cells) |
| Fig. 10 | HeLa (human cervical cancer cells) | Fig. 23 | NCI-H460 (human non-small cell lung cancer cells) |
| Fig. 11 | HepG2 (human hepatoma cells) | Fig. 24 | ACHN (human renal adenocarcinoma cells) |
| Fig. 12 | MDA-MB-231 (human breast cancer) | Fig. 25 | MCF-7 (human breast cancer cells) |
| Fig. 13 | TYK-nu (human ovarian undifferentiated cancer cells) | Fig. 26 | PANC-1 (human pancreatic adenocarcinoma cells) |
| Fig. 14 | HCC2998 (human large intestine adenocarcinoma cells) | Fig. 27 | CFPAC-1 (human pancreatic adenocarcinoma) |
| Fig. 15 | HCT-15 (human colon adenocarcinoma cells) | Fig. 28 | DAUDI (human Burkitt's lymphoma cells) |
| Fig. 16 | HCT 116 (human colon adenocarcinoma cells) | Fig. 29 | HL60 (human promyelocytic leukemi cells) |
| Fig. 17 | LoVo (human colon adenocarcinoma cells (clavicle lymph node)) | Fig. 30 | Jurkat (human T cell leukemi cells) |
| Fig. 18 | SW620 (human colon adenocarcinoma cells) | Fig. 31 | Mono Mac 6 (human monocytic leukemi cells) |
| Fig. 19 | SW837 (human colorectal adenocarcinoma cells) | Fig. 32 | THP-1 (human acute monocytic leukemi cells) |
| Fig. 20 | COLO 205 (human colon adenocarcinoma cells) | | |

Methylation ratio of cytosines of CpG dinucleotides corresponding to the region of SEQ ID NO: 2 (circle 8 to circle 17 in Fig. 7), and methylation ratio of cytosines of CpG dinucleotides corresponding to the region of SEQ ID NO: 1 (circle 6 to circle 21 in Fig. 7), which were calculated based on the results of Figs. 8 to 32, are shown in the following Table 3; and methylation ratio of cytosines of CpG dinucleotides corresponding to the region of SEQ ID NO: 3 (circle 13 to circle 17 in Fig. 7), which were calculated based on the results of Fig. 8 to Fig. 32, are shown in the following Table 4.

**Table 3**

| Example | Cells | Number of methylated cytosine (circle 8 to circle 17) | Methylation ratio | Number of methylated cytosine (circle 6 to circle 21) | Methylation ratio |
|---|---|---|---|---|---|
| Example 1 | hiPS | 1 | 10.0% | 1 | 6.3% |
| | HDF | 2 | 20.0% | 2 | 12.5% |
| | WI-38 | 1 | 10.0% | 2 | 12.5% |
| | CCD-8Lu | 1 | 10.0% | 2 | 12.5% |
| | H23 | 1 | 10.0% | 1 | 6.3% |
| | Whole blood | 1 | 10.0% | 1 | 6.3% |
| Example 2 | IMR-32 | 6 | 60.0% | 8 | 50.0% |
| | HEK293 | 9 | 90.0% | 13 | 81.3% |
| | HeLa | 6 | 60.0% | 8 | 50.0% |
| | HepG2 | 8 | 80.0% | 12 | 75.0% |
| | MDA-MB-231 | 8 | 80.0% | 11 | 68.8% |
| | TYK-nu | 10 | 100.0% | 16 | 100.0% |
| | HCC2998 | 9 | 90.0% | 14 | 87.5% |
| | HCT-15 | 9 | 90.0% | 14 | 87.5% |
| | HCT116 | 10 | 100.0% | 15 | 93.8% |
| | LoVo | 8 | 80.0% | 11 | 68.8% |
| | SW620 | 9 | 90.0% | 14 | 87.5% |
| | SW837 | 10 | 100.0% | 15 | 93.8% |
| | COLO205 | 10 | 100.0% | 15 | 93.8% |
| | WiDr | 9 | 90.0% | 14 | 87.5% |
| | LNCap.Clone FGC | 9 | 90.0% | 14 | 87.5% |
| | NC1-H460 | 9 | 90.0% | 14 | 87.5% |
| | ACHN | 6 | 60.0% | 10 | 62.5% |
| | MCF-7 | 9 | 90.0% | 14 | 87.5% |
| | PANC-1 | 6 | 60.0% | 10 | 62.5% |
| | CFPAC-1 | 9 | 90.0% | 13 | 81.3% |
| | DAUDI | 10 | 100.0% | 15 | 93.8% |
| | HL60 | 10 | 100.0% | 15 | 93.8% |
| | Jurkat | 9 | 90.0% | 13 | 81.3% |
| | Mono Mac6 | 8 | 80.0% | 12 | 75.0% |
| | THP-1 | 8 | 80.0% | 9 | 56.3% |

**Table 4**

| Example | Cells | Number of methylated cytosine (circle 13 to circle 17) | Methylation ratio |
|---|---|---|---|
| Example 1 | hiPS | 1 | 20.0% |
| | HDF | 1 | 20.0% |
| | WI-38 | 1 | 20.0% |
| | CCD-8Lu | 1 | 20.0% |
| | H23 | 0 | 0% |
| | Whole blood | 1 | 20.0% |
| Example 2 | IMR-32 | 3 | 60.0% |
| | HEK293 | 5 | 100.0% |
| | HeLa | 3 | 60.0% |
| | HepG2 | 4 | 80.0% |
| | MDA-MB-231 | 5 | 100.0% |
| | TYK-nu | 5 | 100.0% |
| | HCC2998 | 4 | 80.0% |
| | HCT-15 | 4 | 80.0% |
| | HCT116 | 5 | 100.0% |
| | LoVo | 4 | 80.0% |
| | SW620 | 5 | 100.0% |
| | SW837 | 5 | 100.0% |
| | COLO205 | 5 | 100.0% |
| | WiDr | 4 | 80.0% |
| | LNCap.Clone FGC | 4 | 80.0% |
| | NC1-H460 | 4 | 80.0% |
| | ACHN | 3 | 60.0% |
| | MCF-7 | 4 | 80.0% |
| | PANC-1 | 3 | 60.0% |
| | CFPAC-1 | 5 | 100.0% |
| | DAUDI | 5 | 100.0% |
| | HL60 | 5 | 100.0% |
| | Jurkat | 4 | 80.0% |
| | Mono Mac6 | 3 | 60.0% |
| | THP-1 | 4 | 80.0% |

From the results of Example 1, cells can be determined to be non-cancerous normal cells when methylation ratio of cytosine of CpG dinucleotide present in SEQ ID NO: 2, or SEQ ID NO: 3 is 20% or less. In addition, cells can be determined to be non-cancerous normal cells when methylation ratio of cytosine of CpG dinucleotide present in SEQ ID NO: 1 is 15% or less.

That is, it has been revealed that, when methylation ratio is 30% or more, it can be determined that cells are more likely to be cancerous.

In addition, from the results of Example 2, cells can be determined to be cancerous when methylation ratio of cytosine of CpG dinucleotide present in SEQ ID NO: 2 or SEQ ID NO: 3 is 60% or more.In addition,
cells can be determined to be cancerous when methylation ratio of cytosine of CpG dinucleotide present in SEQ ID NO: 1 is 50% or more. Therefore, it has been revealed that, in consideration of the fact that methylation ratio of cytosine in normal cells is at least less 20% or lower, canceration can be determined in very high precision.

Further, it has been revealed that, even when 25 kinds of different cells are used, by measuring methylation ratio of cytosine of CpG dinucleotide in the same region of the FOXB2 gene, respective canceration can be determined in good precision. That is, it has been revealed that according to the method of the present invention, regardless of the kinds of cancer, determination of cancer can be carried out.

### INDUSTRIAL APPLICABILITY

Using the cancer marker of the present invention, or according to the method of the present invention, it is possible to determine canceration of cells in various cells. In addition, because of measuring methylation ratio of cytosine of the specified CpG dinucleotide, it is possible to determine canceration of cells in good precision. That is, it is possible to be utilized for preventive determination of recurrence or metastasis of cancer.

## Claims

1. A cancer marker consisting of a portion of or all of the nucleotide sequence represented by SEQ ID NO: 1 in a FOXB2 gene derived from a cell, and being continuous nucleotide sequences comprising at least the nucleotide sequence represented by SEQ ID NO: 3, wherein 30% or more of cytosines of the -CG- sequences present in said nucleotide sequence are methylated.

2. The cancer marker according to claim 1 consisting of the nucleotide sequence represented by SEQ ID NO: 3 in the FOXB2 gene derived from a cell, wherein 40% or more of cytosines of the -CG- sequences present in said nucleotide sequence are methylated.

3. A cancer marker consisting of a portion of or all of the nucleotide sequence represented by SEQ ID NO: 1 in a FOXB2 gene derived from a cell, and being continuous nucleotide sequences comprising at least the nucleotide sequence represented by SEQ ID NO: 2, wherein 30% or more of cytosines of the -CG- sequences present in said nucleotide sequence are methylated.

4. The cancer marker according to claim 3 consisting of the nucleotide sequence represented by SEQ ID NO: 2 in the FOXB2 gene derived from a cell, wherein 40% or more of cytosines of the -CG- sequences present in said nucleotide sequence are methylated.

5. A method for determining canceration of cells, wherein methylation ratio for a portion of or all of cytosines of the -CG- sequences present in the nucleotide sequence represented by SEQ ID NO: 1 in a FOXB2 gene derived from a cell, is measured by a bisulfite reaction, and canceration of the cell is determined on the basis of methylation ratio.

6. The method according to claim 5, wherein when methylation ratio is 30% or more, the cell is determined to be cancerous.

7. The method according to claim 5, wherein methylation ratio of all of cytosines of the -CG- sequences at least in the nucleotide sequence represented by SEQ ID NO: 3, is measured.

8. The method according to claim 7, wherein when methylation ratio is 40% or more, the cell is determined to be cancerous.

9. The method according to claim 5, wherein methylation ratio of all of cytosines of the -CG- sequences at least in the nucleotide sequence represented by SEQ ID NO: 2, is measured.

10. The method according to claim 7, wherein when methylation ratio is 40% or more, the cell is determined to be cancerous.


SEQUENCE LISTING

<110>  Wako Pure Chemical Industries, Ltd.

<120>  Cancer marker and the method for determining cancer

<130>  1961JP

<160>  9

<170>  PatentIn version 3.5

<210>  1
<211>  150
<212>  DNA
<213>  Human FOXB2

<400>  1
cgggggaaga cgcagagaag gcggcggtaa acctggtgca ctccgcccgc gactgtgcgc      60

gccagtcggc aaccgtcggg gccagaagtt gccagcttcc gagagctgag taggcccgaa     120

aggccaaggt cggagacacc aggcaattcg                                      150


<210>  2
<211>  57
<212>  DNA
<213>  Human FOXB2

<400>  2
cggcggtaaa cctggtgcac tccgcccgcg actgtgcgcg ccagtcggca accgtcg        57


<210>  3
<211>  21
<212>  DNA
<213>  Human FOXB2

<400>  2
CGCGCCAGTC GGCAACCGTC G                                               21


<210>  4
<211>  26
<212>  DNA
<213>  Artificial Sequence

<220>
<223>  Primer

<400>  4
ggtttggtta agtgtttggt tagggg                                          26


<210>  5
<211>  28
<212>  DNA
<213>  Artificial Sequence

<220>
<223>  Primer

<400>  5
gtagttagtt ttagtattta gtttttgg                                          28


<210>  6
<211>  27
<212>  DNA
<213>  Artificial Sequence

<220>
<223>  Primer

<400>  6
cctaactacc cccactaatt cccactc                                           27


<210>  7
<211>  29
<212>  DNA
<213>  Artificial Sequence

<220>
<223>  Primer

<400>  7
ccctctctac ttctctctcc taccttctc                                         29


<210>  8
<211>  39
<212>  DNA
<213>  Artificial Sequence

<220>
<223>  Primer

<400>  8
ttaccataag cttggtttgg ttaagtgttt ggttagggg                              39


<210>  9
<211>  40
<212>  DNA
<213>  Artificial Sequence

<220>
<223>  Primer

<400>  9
taattaagga tcccctaact acccccacta attcccactc                             40


<210>  10
<211>  493
<212>  DNA
<213>  Human FOXB2

<400>  10
ggtctggcta agtgcctggc caggggcctg cgtccagggc actggaagtc ctgctgtcac      60

tggcctccag gacagcaggc gcaaactcag atttaaaggg ccagagtctc tactgcccag     120

accttcctcc cagccatctt ggtctgggtt tccctcggac ctagaaaaag caaagaggcc     180

gggaggaaac acggcccctt gccactctcc tctgcagcca gctccagcac tcagtctttg 240

gccacccggg ggaagacgca gagaaggcgg cggtaaacct ggtgcactcc gcccgcgact 300

gtgcgcgcca gtcggcaacc gtcgggccca gaagttgcca gcttccgaga gctgagtagg 360

cccgaaaggc caaggtcgga gacaccaggc aattcggaga aggcaggaga gagaagcaga 420

gagggcctgg agggcgagag ggcaaagtgg cgggactgga ggggccgagt gggaattagt 480

gggggcagcc agg 493